# EUROPEAN PATENT APPLICATION

(11) **EP 1 842 584 A1**
(43) Date of publication of application: **10.10.2007**
(21) Application number: 06708852.6
(22) Date of filing: 26.01.2006
(51) Int. Cl.: B01J 13/04, B05B 7/06, A61K 9/50, A61K 9/51

(54) **METHOD AND DEVICE FOR OBTAINING MICRO AND NANOMETRIC SIZE PARTICLES**

(30) Priority: 28.01.2005 ES 200500205
(71) Applicant: Universidad de Sevilla, E-41012 Sevilla (ES)
(72) Inventor: GAÑÁN CALVO, A.M., E.T.S. Ingenieros Industriales, de los Descubrim. s/n, 41092 Sevilla (ES); MARTÍN BANDERAS, L E.T.S. Ingenieros Industriales, de los Descubrim. s/n, 41092 Sevilla (ES); FLORES MOSQUERA, M. E.T.S. Ingenieros Industriales, de los Descubrim. s/n, 41092 Sevilla (ES); RODRÍGUEZ GIL, Alfonso, Dpto. Genética, 41012 Sevilla (ES); CHÁVEZ DE DIEGO, Sebastián, Dpto. Genética, 41012 Sevilla (ES); CEBOLLA RAMÍREZ, Ángel, Dpto. Genética, 41012 Sevilla (ES)
(74) Representative: Elzaburu Marquez, Alberto
(86) International application number: PCT/ES2006/000033
(87) International publication number: WO 2006/082263

(57) **Abstract**

The invention relates to a method and device for obtaining micro and nanometric particles in a controlled, reproducible manner. The aforementioned particles have a spherical shape and a very narrow, uniform size distribution. More specifically, the invention relates to a novel method of forming emulsions and to the application thereof in micro and nanoencapsulation techniques involving the extraction/evaporation of the solvent. In particular, the invention relates to the encapsulation of the fluorescent compounds and the subsequent application thereof.

## Description

### AIM OF THE INVENTION

The hereby-presented invention is concerning the generation of polymeric particles in the micro and nanometric range with a controlled and reproducible method. Said particles have spherical shape and a very narrow and homogeneous distribution. Particularly, the present invention describes a new method for the production of emulsions and its application to micro and nanoencapsulation by means of solvent extraction/evaporation. Specially, the hereby-presented invention refers to the encapsulation of fluorescent compounds and their application.

### STATE OF THE ART

Currently, the use of microparticles has spread widely in very diverse fields such as pharmacy, biomedicine, cosmetic industry, food industry, agriculture, veterinary science, textile industry, chemistry, etc. Among others, the most interesting application is the possibility to use microparticles as a method to stabilize and protect products from the environment and/or as procedure to optimize the distribution/way of an encapsulated compound in its way to the application/interaction point.

One of the fields where the use of microparticles is acquiring special prominence is genomic and proteomic sciences where they are being used as a new method to study biomolecular interactions. Despite the enormous advances occurring in the production and application of microarrays of diverse nature, traditional microarrays show some disadvantages not yet solved. One of the main problems is the complexity of the protocols for sample preparation, which are also rather slow, and the increase of costs of the technology used to analyze the results. The reproducibility and reliability of the obtained results are still not fully achieved by traditional arrays.

Currently, the use of arrays of fluorescent microparticles with a modified surface is taking advantage to the use of traditional 2D microarrays. Generally, microparticles allow carrying out multiple simultaneous tests, they are cheaper, easier to store even in big amounts and have a high usable surface per volume unit for molecule coupling. In addition, the microspheres can be produced in different sizes and chemical composition, giving high versatility in their application in biological tests performed with molecules of diverse nature.

Another big advantage of using arrays of microparticles is that they allow the use of flow cytometry as detection technique for the molecular interactions that take place. Flow cytometry is a widely spread technique which has been recently undergoing a great evolution increasing considerably the speed of detection, sensitivity and reliability of the analysis and is therefore substituting other techniques for the analysis of complex samples tests. The development of more and more sophisticated analysis equipments with a high sensitivity, together with the improvement of more and more versatile techniques for production of microparticles, have aroused big expectation for the future both in basic research and in clinical diagnosis.

On the other hand, the application of encapsulated fluorophores show many advantages compared to the application of independent organic molecules or fluorescent nano-crystals. Fluorophores are inside a matrix that protects them from the effects of solvents, pH, ionic forces, photobleaching, etc. stabilizing the intensity of the emission. Moreover, the surface of the particle remains free with the functional groups for conjugation with proteins, nucleic acids or other type of biomolecules, that are allocated outside the particle without affecting the fluorescent qualities of the dye. Therefore, the application of bioconjugated fluorescent microparticles allows to increase significantly the sensibility of detection avoiding aggregation or inactivation due to the excess of fluorescencent dye.

There are a number of encapsulation techniques that may vary according to the fluorescent material that we try to encapsulate. Among them the most used ones are: ■ absorption/diffusion of organic fluorophores or fluorescent nanocrystals inside of a microparticle through a polymeric matrix (e.g. Han, M.Y.; Gao, X.; Su, J.Z.; Nie, S. "Quantum-dot-tagged microbeads for multiplexed optical coding of biomolecules" Nat Biotechnol 2001, 19 631-635; US Pat Nr 6680211, Biocrystal Ltd.; US Pat Nr 5723218, Molecular Probes; L.B. Bangs, Uniform Latex Particles, Seragen Diagnostics Inc, 1984; US Pat Nr 6649414, Luminex Corp. where they also describe the covalent junction of fluorescent nanoparticles obtained by absorption of fluorophores to bigger size microparticles); the incorporation of fluorescent material during the formation of the microparticle in the polymerization and/or covering process, etc.(e.g. US Pat Nr 5073498, Caribbean Microparticles Corp.; W00113119, Luminex Corp.); ■ the layer-by-layer chamberion of polymer upon the fluorescent material (e.g. Wang, D.; Rogach, A.; Carusso, F. Semiconductor quantum dot-labeled microsphere bioconjugates prepared by stepwise self-assembly. Nano Lett 2002, 2 857-861).

The chamberion of layers of polymer upon the fluorescent material implies the production of the particle "in situ" and requires a high control above each step of the process in order to obtain particles of the requested size with a high degree of homogeneity. It is a laborious process implying numerous steps.

In the case where the fluorescent material is introduced inside the particle by diffusion, the particle is already produced but it is necessary to have a big control over the homogeneity of the initial fluorophore solution and the required time to obtain the specific load of each fluorosphre. One of the main disadvantages of this method is the lack of homogeneity in the final distribution of the fluorophores inside the microparticle so the final concentration of the fluorescent material in the surface is rather high and decrease gradually when approaching the core. Besides, although the fluorophores are protected inside the matrix, in the case of particles generated by diffusion, there is a high percentage of fluorophores in the surface (approximately 30%) showing different characteristics to those embedded inside the matrix that indeed could produce agglomerates and loss of photoluminescence of the final microparticles.

Finally, the incorporation of fluorescent material during the formation of the microparticle is a process that generally implies the growth of an initial particle by means of the polymerization of a monomer in the presence of a fluorophore, being no better alternative than the previous ones.

Considering everything previously said, it is necessary the application and optimization of new encapsulation methods to produce codified particles in a simple way, allowing to control exhaustively the structure and homogeneity of the particle, as well as distribution of fluorescent materials used for its coding.

### DESCRIPTION OF THE INVENTION

This patent refers to a new system for particle production based in the generation of emulsions by means of a Flow Focusing system, which combines the application of hydrodynamic forces and a specific geometry of the system. After the solidification of the drops we obtain particles of nano and micrometric size, with a spherical shape and a very narrow and reproducible size distribution.

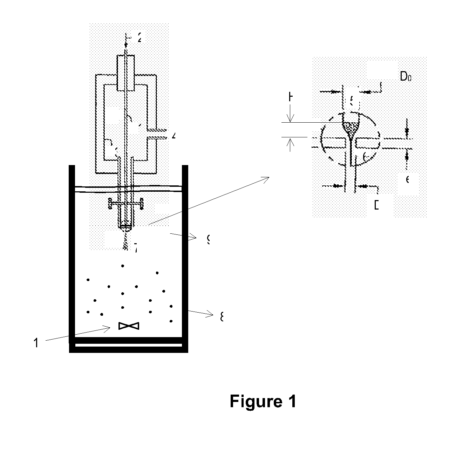

The system for production of particles aimed by this invention is constituted by a flow focusing device immersed in a liquid that will become the external phase of the produced emulsion. To be precise, the flow focussing device consists of (Fig 1) a chamber pressurized by means of the continuous supply of a fluid. Inside the chamber a second fluid is injected through a feeding point placed in front of a hole made on the wall of the chamber. The fluid flow pressurizing the chamber surrounds the second fluid that is expelled outside the chamber through the hole producing a thin microjet in a controlled way.

Due to capillary instability the capillary microjet located inside the laminar flow breaks inside the liquid wherein the device is immersed, producing a homogeneous emulsion with controlled sized drops. Once the emulsion has been produced and after solidification of the drops, we obtain dry particles with spherical shape. In this procedure both described fluids are preferably liquids, different enough between themselves to allow the generation of a stable microjet. Generally, the inner fluid is a solution of one or more components, a liquidized solid, a suspension and/or an emulsion of compounds of diverse nature.

In the first procedure, one single fluid is injected through one single feeding tube inside the chamber pressurized by the second fluid, and is then expelled to the outside through a hole placed in the wall in front of the end of the feeding tube.

In the second procedure included in this invention, the injected fluid consists of different fluids, which are introduced through concentric capillary tubes. Inside the chamber, these fluids get in touch creating a capillary microjet formed by several layers of concentric fluids. This capillary microjet is pressurized and expelled to the outside by the liquid pressurizing the chamber through the hole placed in the wall in front of the end of the feeding tubes.

It is aim of this invention the use of multiple fluid feeding tubes inside the pressurized chamber, producing multiple microjets that exit to the outside through the holes made in front of each feeding tube. It is the aim of this invention the device and the procedures for the generation of multiple capillary microjets in order to produce emulsions, and later on particles in big amounts.

As an option, it is included in this invention the possibility to apply periodical and controlled external instabilities (e.g. mechanical, acoustic, etc.) to one or more fluids, in order to enhance even more the production of particles with a homogeneous size distribution.

It is the aim of this invention to provide a new method for producing dry particles of predictable and controlled size with a very low dispersion rate. It is included in this invention a process for encapsulation of different substances inside the particles. Mainly, it is included in this invention a process for encapsulation of fluorescent materials in a controlled way in one single step.

It is also included in this invention a method for producing particles containing molecules of biological interest exposed to the external environment of the particle.

Another aim of this invention is to produce particles that can be used as calibration standards (size, fluorescency, etc.), in the production of arrays of microparticles, in pharmaceutical and biomedical applications (encapsulation and drug delivery, cells and microorganisms encapsulation, diagnosis and clinical analysis) etc.

Main innovative and advantageous characteristics of this combination of geometry and hydrodynamic effect compared to any other existent methodology are:
a) Particles of much smaller size than any other dimension of the device (hole, feeding end, etc.) can be produced, what is not possible by means of other existent extrusion techniques that force the size of the particle by means of the injection hole.
b) The size of the particle is controllable and predictable.
c) Particles size distribution is very narrow without using more complex mechanisms to manipulate the jet or to perform a later selection process.
d) It is a fully reproducible process.
e) There is no contact between the internal fluid and the exit hole, thus preventing problems with the great shear stresses associated to direct extrusion. It also prevents clogging.
f) The formation of the particles takes place inside the external phase of the generated emulsion preventing deformation problems in the final geometry of the particle.
g) It allows the use of an enormous combination of fluids with diverse physico-chemical characteristics (viscosity, chemical composition, etc.).
h) Final particle composition and structure is fully controllable from the very start of the process.
i) It is a system for production of particles in a continuous way, which allows to carry out exhaustive quality controls in each step of the production process.

### DESCRIPTION OF THE FIGURES

Figure 1: general scheme with basic components of a device for the generation of particles by means of injection of one single fluid according to the hereby described procedure.
Figure 2: general scheme with basic components of a device for the generation of particles by means of the injection of two concentric fluids.
Figure 3: chart depicting d₅₀ part/do to Q/Q₀ for some performed experiments and their agreement to Flow Focussing predictions.
Figure 4a: electron microscope photography of 5 micron polystyrene particles produced by means of the procedure described in this invention.
Figure 4b: electron microscope photography of 5 micron polystyrene particles produced by means of the procedure described in this invention.
Figures 5a-c: fluorescence microscope images of a mixture of 5 micron polystyrene fluorescent particles in two different and differentiated concentrations of rhodamine produced by means of the procedure described in this invention. Fig. 5a: concentrations of rhodamine 0,006 mM and 0,6 mM. Fig. 5b: concentrations of rhodamine 0,06 mM and 0,6 mM. Fig. 5c: concentrations of rhodamine 0,006 mM and 0,06 mM.
Figure 6: fluorescence microscope images of a mixture of 5µm polystyrene fluorescent particles with a concentration of fluorescein (1mM) and rhodamine B (0,6 mM). Fig. 6a: uses a filter (L5) where the two particle populations can be seen.
Fig. 6b: uses a filter (N3) where only the particles containing rhodamine B can be seen.
Figure 7: chart of the flow cytometry analysis of a mixture of polystyrene particles without fluorescence, with rhodaine B and with fluorescein produced by means of the procedure described in this invention.

### DETAILED DESCRIPTION OF THE INVENTION

Prior to the description of the method and device aim of this invention, it is to be understood that this invention is not restricted neither to the specific components nor to the concrete procedures hereby described, as such may, of course, vary. In general, it is meant that all terminology used here, is used to describe the different aspects of the invention and does not intend to be in any case limiting.

Devices used for the achievement of this invention are described in the documents WO9930833 ("Device and method for creating dry particles"), WO9930834 ("Device and method for creating aerosols for drug delivery"), US6234402 ("Stabilized capillary microjet and devices and methods for producing the same").

In general and except where stated, all scientific and technical terms used herein have the same meaning that is usually understood when applied in related environments.

### Definitions

It must be noted that as used herein and in the appended claims, the singular forms "a", "one", and "the", can also indicate plurals unless the context indicates otherwise. For example, when it is written "a particle" it includes a group of particles and when it is referred to "a fluorophore" it includes a combination of fluorophores, etc.

In this invention the terms *sphere, particles and capsules* are interchangeable for the description of the micro and nanoparticles described in the patent, regardless of whether they are solid, hollow, porous, with different layers o multi-layer, etc. These particles can be made of any material depending on the final application.

With the term fluorescent material we refer to any type of material emitting a fluorescent signal, whether they are organic compounds, biomolecules, nanocrystals, nanoparticles, liquids, solids, etc. individually or as a mixture of several of them.

With the term *reactive surface* we refer to the surface of the particles that, having any composition, have a series of functional groups that may react with any type of molecule containing the appropriate chemical functionality making possible the creation of one or more covalent links between particle and molecule.

In this invention the term *fluid* is used without distinction to name gases or liquids. In the case of liquids it includes simple liquids, mixtures, dissolutions, suspensions, emulsions, liquidized solids, etc.

With the term *microjet* we refer to the capillary filament obtained from the focussing of the internal fluid inside the pressurized chamber that exits to the outside through a hole performed in the chamber. This term includes jets with nano and micrometric diameter and of different composition.

### Description of the invention

The aim of this invention is the production of particles based on a new methodology for generation of emulsions. This new procedure is based on a capillary focussing system, named Flow Focussing, which combines the application of hydrodynamic forces and a specific geometry of the system and that takes place inside the external phase of the future emulsion. Once the emulsion has been produced and after solidification of the drop we obtain particles of nano and micrometric size, with spherical shape and a very narrow and reproducible size distribution.

The basic technology of the invention lies on a system for introduction of a first fluid inside a pressurized chamber by means of a second fluid. The first fluid can be a liquid or gas and the second fluid is a liquid. Preferably, both fluids are liquids different enough one to another to allow the generation of a stable microjet of the first fluid moving from the feeding end to the exit point of the pressurized chamber to the environment. Considering both possible combinations gas-liquid and liquid-liquid, this invention will describe the general procedure for the liquid-liquid combination.

The formation of the microjet, its acceleration and finally the generation of the particles is based on the abrupt pressure drop associated to the sudden acceleration undergone by the first fluid pressurizing the chamber when going through the exit hole of the chamber. This causes a high pressure difference between both fluids, which originates the appearance of a high curve area in the surface of the second fluid close to the hole and obtaining a peak point from where a stationary microjet will flow if we provide the same amount of liquid than the hole sucks. Thus, the fluid in the pressurized chamber surrounds the first fluid focussing it, generating a stable microjet whose diameter is much smaller than the diameter of the exit holeof the chamber preventing its clogging.

We use a parametric space (properties of the fluids, supplied flow rates, feeding end diameter, size of the exit hole, pressure relationship, etc.) wide enough to be applied to almost every liquid (dynamic viscosities from 10⁻⁴ to 1 Kg.m-¹-s-¹), so the capillary microjet rising from the feeding tube is absolutely stable and the instabilities in the microjet dissociation process cannot develop upstream.

Downstream the microjet breaks into drops of regular size simply by capillary instability (e.g. Rayleigh "On the inestability of jets" Proc. London Math. Soc, 4-13, 1878).

The microjet is generated inside the fluid in motion in a way that when the microjet breaks into similar drops of the predicted size the emulsion is produced. After extraction/evaporation of the solvent by means of any of the already known methods we obtain particles of nano- and micrometric size with spherical shape and a very narrow and reproducible size distribution, as predicted by Flow Focussing theory.

It is an aim of this invention a system composed of a device for the generation of drops and a container for collecting those drops where the external phase of the emulsion is placed. The system is composed of (Fig. 1):
1. Feeding tube, also called feeding source or feeding needle or tip or end or feeding end.
2. Beginning of the feeding tube used to introduce the internal fluid.
3. Pressurized chamber.
4. Orifice used as fluid pressurizing inlet.
5. End of the feeding tube that introduces the first fluid in the pressurized chamber.
6. Orifice through which withdrawal takes place.
7. Microdrops.
8. Container wherein the produced drops are collected.
9. Liquid wherein the nebulizer for the generation of the drops is immersed and that will become the external phase of the emulsion.
10. External system for shaking the emulsion while being generated.

D₀ is the diameter of the feeding tube; D is the diameter of the orifice through which the microjet is passed; e is the axial length of the orifice through which withdrawal takes place; H is the distance from the feeding tube to the microjet outlet; P₀ is the pressure inside the chamber; P_{α} is atmospheric pressure.

Although the device included in this invention can be configured in a variety of designs, the different designs will all include the essential components shown in Fig. 1, or components which perform an equivalent function and obtain the desired results. Specifically the device of the invention will be comprised of at least one feeding source (1) for the internal fluid open on both ends, one (2) for introduction of the internal fluid and the other (5) to expel it in the pressurized chamber (3) with 0,002-2 mm inner diameter, preferably 0,01-0,4 mm. The feeding tube (1) or at least the exit end (5) must be inside the pressurized chamber (3). This chamber (3) must have an inlet opening (4) which is used to feed a second fluid into the chamber (3) and an exit opening (6) with 0,002-2 mm inner diameter, preferably 0,01-0,25 mm, through which the capillary microjet will flow. The exit end (5) of the feeding tube (3) must be in front of the exit hole of the chamber and at a distance of 0,01-2 mm, preferably 0,2-0,5 mm.

Additionally it is aim of this invention a device where the diameter of the tip of the feeding source, the diameter of the orifice of the chamber and the distance between them can be changed and adjusted in order to obtain conditions for the interaction between the fluids leading to a stable capillary microjet inside a laminar flow.

The introduction of the fluids will take place by means of any method allowing a continuous supply of fluids without fluctuations of the flow rate (compressors, pressurized chamber, volumetric pumps, etc.).

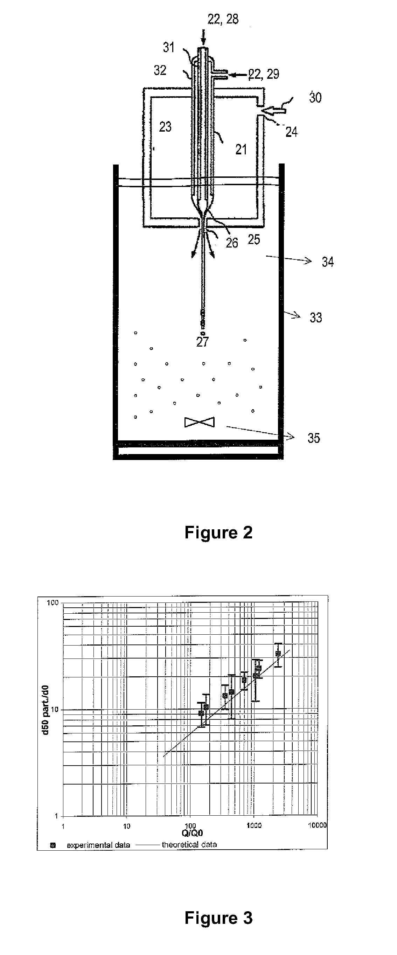

In Fig. 1 the feeding source and the pressurized chamber are designed to obtain emulsions where the drop size is small and has a uniform distribution. According to this invention, the extraction/evaporation of the solvent takes place producing only a reduction in the volume of the particle due to the solvent elimination. This elimination happens rapidly with no coalescence phenomenon of the drops, clogging or similar taking place, thus keeping the relative size distribution of the drops on final particles. The size of the particle obtained agrees to the size predicted by Flow Focussing theory (Fig. 3). In some cases, sizes slightly bigger to those foreseen are obtained, because a slowing down and widening of the focussed flow microjet takes place before it breaks up due to the deceleration undergone by the external jet of the focussing fluid.

Preferably final particles should have diameters between 0,01 and 1000 µm, more preferably between 0,01-200 µm and most preferably between 0,01-80 µm. Final particles obtained should be equal in size with a relative standard deviation of 10 to 30 %, more preferably 3 to 10 % and most preferably 3 % or less.

As an option, it is included in this invention the possibility to apply periodical and controlled external perturbations (e.g. mechanical, acoustic, etc.) to one or more fluids in order to enhance even more the production of particles with a homogeneous size distribution. These instabilities must be uniform and controlled and their frequency will be determined by the characteristics of the generated microjet and the required final particle size.

It is the aim of this invention a Flow Focussing device for the production of multiple capillary microjets by means of the use of multiple fluid feeding tubes placed each of them in front of one of the multiple orifices located in the pressurized chamber wall. For obtaining optimal results in the case of multiple devices, the fluid flow rate to be injected should be as homogeneous as possible in every point, the introduction of the fluid can take place by means of multiple capillary needles, porous media or any other medium able to distribute an homogeneous flow rate among different feeding tubes.

This nebulizer can be manufactured in multiple materials (metal, plastic, ceramics, glass, etc.) depending basically on the specific application in which the device will be used.

It is another aim of this invention a device whose design includes a plurality of feeding needles placed concentrically to each other producing at the exit one single capillary microjet (Fig. 2). The components of the embodiment of Fig 2 are as folows:
21. Feeding needle, also called tube or source of fluid or feeding end.
22. Beginning of the feeding tube used to introduce the fluids in the feeding source.
23. Pressurized chamber.
24. Entry orifice used as pressurizing fluid inlet.
25. End of the feeding tube that introduces the first fluid in the pressurized chamber to be atomized.
26. Orifice though which the microjet exits the pressurized chamber.
27. Microdrops.
28. Fluid that will constitute the core of the particle.
29. Fluid that will constitute the outer coating of the particle.
30. Fluid pressurizing the chamber.
31. Internal capillary of the feeding source.
32. External capillary of the feeding source.
33. Container where the produced drops are collected.
34. Liquid wherein the nebulizer for the generation of the drops is immersed and that will constitute the external phase of the emulsion.
35. External system for shaking the emulsion while being generated.

Dᵢ is the diameter of the internal capillary of the feeding tube; D₀ is the diameter of the external capillary of the feeding tube; is the diameter of the orifice through which the microjet is passed; e is the axial length of the orifice through which withdrawal takes place; H is the distance from the feeding tube to the microjet outlet; P₀ is the pressure inside the chamber; P_{α} is atmospheric pressure.

The device included in this invention can be designed in multiple ways, so that it still contains the basic components shown in Fig. 2, or components achieving the same function getting to the desired results.

The system described in Fig. 2 is used when we want to obtain a substance coated by one or more different substances. This system is composed of the same basic component as the one described in Fig. 1 and further includes a second feeding source which is positioned concentrically around the first cylindrical feeding source. The external capillary may be as well surrounded by new capillaries placed concentrically around the previous ones.

The procedure for the production of emulsions is the same than the previous one, so that the fluids are injected separately through a special feeding source (21) made out of concentric capillaries. If the particles consist of two materials, through the internal capillary (31) is injected the material that will constitute the nucleus/core of the particle and through the external capillary (32) is introduced the material that will coat the particle. Both feeding tubes have 0,002-2 mm inner diameter (preferably 0,01-0,4 mm), being Dᵢ<Dₒ. Also the relative distance between the tips of the concentric tubes can change taking into account that the internal tube (31) must not enter the external tube (32) a distance higher than the value of said external tube inner diameter (32). These same criteria are valid if we use two concentric capillaries keeping those relative positions between consecutive tubes.

At the exit of the feeding source (25) the fluids join concentrically inside the pressurized chamber, they are accelerated by the fluid pressurizing the chamber producing a microjet of two or more concentric layers of different fluids, at least not during the generation and break up of the microjet process. The feeding source is placed in front of the exit hole of the chamber, which has 0,002-2 mm inner diameter (preferably 0,01-0,25 mm), and at a distance of 0,01-2 mm (preferably 0,2-0,5 mm) from the hole. Preferably the distance between the feeding end of the internal capillary and the chamber orifice must be included between zero and three times the value of the external capillary inner diameter. Once the microjet has been generated and due to capillary instability, it breaks producing spherical drops of similar size homogeneously and that will be constituted by more or less concentric layers of the different fluids. Size and thickness of the different layers constituting the microparticle and their relative distribution are determined by the relationship among flow rates of the different fluids and the relationship of their feeding capillaries inner diameters and their relative positions, which can be adjusted precisely.

Additionally it is an aim of this invention a Flow Focussing device where the diameter of the tip of the feeding source, the diameter of the exit hole of the chamber and the distance between them can be changed and adjusted in order to obtain interaction conditions between the fluids leading to a stable capillary microjet inside the laminar flow.

The introduction of the fluids will take place by means of any method allowing a continuous supply of fluids without fluctuations of the flow rate (compressors, pressurized chamber, volumetric pumps, etc.).

In Fig. 2 the feeding source and the pressurized chamber are designed to obtain emulsions where the drop size is small and has a uniform distribution. According to this invention, the extraction/evaporation of the solvent takes place producing only a reduction in the volume of the particle due to the solvent elimination. No coalescence phenomenon of the drops, clogging or similar, take place, thus keeping the drops to final particles relative size distribution. Preferably final particles should have diameters between 0,01 and 1000 µm, more preferably between 0,01-200 µm and most preferably between 0,01-80 µm. The obtained final particles should be equal in size with a relative standard deviation of 10 to 30 %, more preferably 3 to 10 % and most preferably 3 % or less.

As an option, it is included in this invention the possibility to apply periodical and controlled external perturbation (e.g. mechanical, acoustic, etc.) to one or more fluids in order to enhance even more the production of particles with a homogeneous size distribution. These perturbations must be uniform and controlled and their frequency will be determined by the characteristics of the generated microjet and the required size of the final particle.

It is the aim of this invention a device for the generation of multiple capillary microjets by means of the use of multiple fluid feeding tubes, each of them constituted by two or more concentric capillaries, placed each of them in front of one of the multiple holes located in the pressurized chamber wall. To obtain optimal results in the case of multiple devices, the fluid flow rate to be injected should be as homogeneous as possible in every point, the introduction of the fluid can take place by means of multiple capillary needles, porous media or any other medium able to distribute an homogeneous flow rate among different feeding tubes.

This nebulizer can be manufactured in multiple materials (metal, plastic, ceramics, glass, etc.) depending basically on the specific application in which the device will be used.

The nature and composition of the fluids referred to in this invention depend on the composition and structure of the particles and the final application they are produced for. In this invention the term *fluid* is used without distinction to name gases or liquids. In the case of liquids it includes simple liquids, mixtures, dissolutions, suspensions, emulsions, liquidized solids, etc.

Taking into account the final composition and structure of the particle, we must use a combination of fluids that: - has the required properties for the generation of a stable capillary microjet by means of any of the capillary flow focussing devices included in this patent, and - generates a capillary microjet that breaks up producing a stable emulsion which allows, after solidification of the drop, obtaining particles of the predicted size and with a homogeneous size distribution. For example and with no restrictive intention, for the generation of simple particles from a hydrophobic material, both the pressurizing material and the dissolution where the generation of the drops takes place, will be hydrophilic solutions and not miscible with the focussed fluid, in order to generate an emulsion that, after extraction/evaporation of the solvent, produces the expected particles.

The particles can be produced in different materials, including but not limited to polymers, silica, metal, ceramics, etc. This invention includes preferably polymeric materials. Polymers can be synthetic or natural, soluble in water or in organic solvents. Aims of this invention are fluids containing polymeric materials among which we can include, without limiting this invention: polyalcohols, polyacetals, polyethers, polyesters (such as polylactic acid, polyglycolic acid, poly(caprolactone) and similar ones and their copolymers), polyorthoesters, polyanhydrides (such as polysebacic acid, polyfumaric acid, poly(carboxyphenoxy propane), poly(carboxyphenoxy hexane) and similar ones and their copolymers), polyaldehydes, polyketones, polycarbonates, poly(iminocarbonates), polyamides, polyimide, polyacrylates and their derivates and copolymers, poly(cyancrilates), polyurethanes, polystyrenes, polychlorides, polyfluorides, polyvinyl derivates, polyolefins, polyphosphates, poly(organic phosphacens), poly(anhydrides-co-imides), polysaccharides, and carbohydrates derivates, poly(aminoacid), polymers derived from macromolecules, and all derivates of the ones mentioned above and their copolymers.

It is aim of the invention that polymeric materials used in the production of particles have functional reactive groups that may react with any type of molecule containing the appropriate chemical functionality making possible the creation of one or more covalent links between particle and molecule. Preferably it is aim of this invention that those reactive groups are located in the surface of the particle directed to the outer surface of the particle. It is included in this invention that among the molecules link to the surface are included, but not limited to, molecules of biological interest, preferably peptides, oligonucleotides, nucleic acids, PNAs, LNAs, proteins, glycoproteins, lipids, phospholipids, carbohydrates, oligosaccharides and mixtures of those.

Also it is included in this invention a method for the generation of particles with which it is possible to add in one single step of the production process molecules of biological interest directed to the exterior of the particle.

Besides the main components that will constitute the particle matrix or matrixes (in the case of multilayer capsules), fluids can be composed of other substances among which they are included, but not limited to: drugs and compounds with therapeutic and/or prophylactic activity, proteins, microorganisms, cells, biomolecules, substances with biological activity in animal world, metals, substances with magnetic properties, colourings, fluorophores, etc.

Preferably it is included in this invention any type of substance emitting a fluorescent signal, whether they are organic compounds, biomolecules, nanocrystals, nanoparticles, liquids, solids, etc. individually or as a mixture of several of those. Predominantly, it is included in this invention any type of fluorescent material that besides being used individually, can be used in combination with others in the same particle, characterized by: - having an excitement spectrum in the same range of wave lengths and, - having an emission spectrum that enables to distinguish them when used simultaneously. Explicitly it is included in this invention those fluorescent materials that constitute a homogeneous mixture (solution, suspension, emulsion, etc.) with the fluid where they will be injected during the production process of the emulsion. The control of both, the fluorescent material composition in the injection mixture and the conditions for drop production allow obtaining particles of the desired size and with the required fluorescent properties, predictable and differentiated. Codified fluorescent particles can be analyzed and differentiated by means of habitual technology (spectrometer, fuoroscency microscopes, etc.), and preferably flow cytometry.

It is also aim of this invention that the particles containing fluorescent material inside them have in their surface functional reactive groups able to create covalent links between the fluorescent particle and other molecules.

It is aim of this invention that during the emulsion production process the device for the generation of drops is immerse in a fluid that will constitute the external phase of the emulsion so the drops don't undergo any deformation process during the generation of the emulsion. This fluid is a liquid that can have aqueous or organic nature, and whose properties are different enough from the fluid or fluids constituting the drops in order to produce an emulsion with droplets equally in size to those produced by the dissociation of the capillary jet. Also, the fluid wherein the device is immersed can have substances in solution which enhance the generation and maintenance of uniformity and homogeneity of the emulsion during the drop solidification process for obtaining the particle (surfactant, emulsifying, tensioactive, etc.). Additionally, the fluid wherein the emulsion generates is in motion.

It is included in this invention a procedure for the generation of emulsions with very diverse characteristics according to the nature of the fluids in use (e.g. w/o, o/w, o/o, w/o/w, w/o/o, etc.).

Besides it is included in this invention the methods for extraction/evaporation of solvent usually used for the production of particles from emulsions without loss of their initial properties, with no coalescence processes, clogging, etc that should change modify morphology and size distribution of the particles.

Additionally, this invention has as preferred procedure the one by means of which the generation of the particle with a reactive surface and the encapsulation of the fluorescent material takes place in one single step, using one of the devices described and included in this invention. The generation of fluorescent particles takes place by means of the injection of the homogeneous mix, constituted by the encapsulating material and the fluorophore or combination of fluorophores, through the feeding tube inside the pressurizing chamber; the generation of a stable capillary microjet due to suction originated by the pressure change undergone by the pressurizing fluid when exiting the pressurized chamber through the chamber orifice, which is located opposite to the feeding point of the injected fluid; the axial-symmetric dissociation of the capillary microjet inside the fluid wherein the device producing the emulsion is immersed; and finally, the extraction/evaporation of the solvent to produce the fluorescent particles. To the surface of these codified fluorescent particles we link covalently molecules of biological interest.

Another aim of this invention is to produce particles that can be used as calibration standards (size, fluorescence, etc.) in the production of arrays of microparticles, in pharmaceutical and biomedical applications (encapsulation and delivery of drugs, cells and microorganisms encapsulation, diagnosis and clinical analysis) etc. Preferably this invention includes the production of arrays of codified fluorescent particles modified superficially in order to use them in biological multiplex tests.

### METHOD FOR ACHIEVEMENT OF THE INVENTION

### EXAMPLE 1

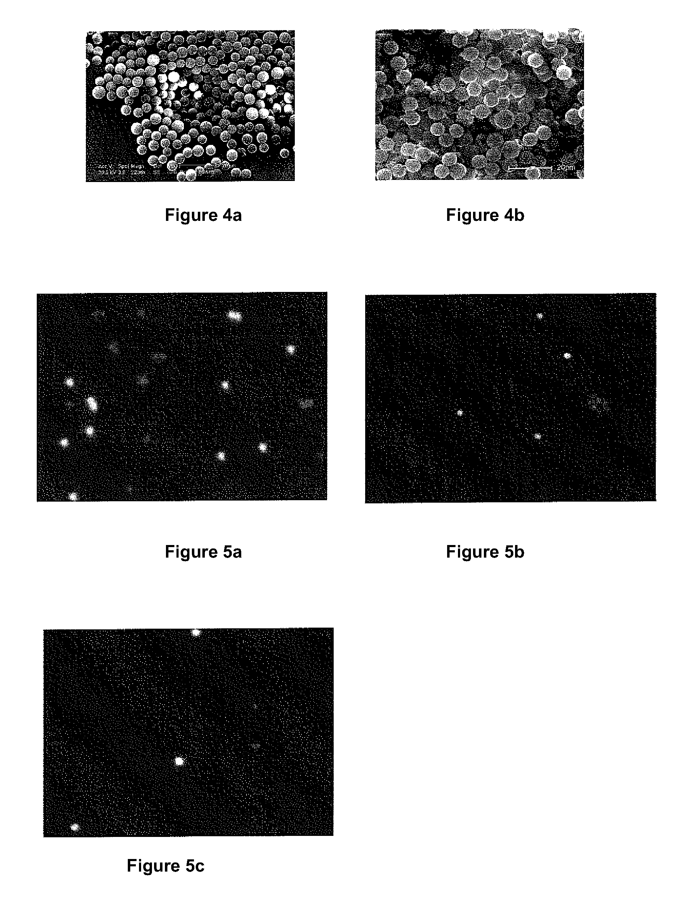

### Preparation of 5 micron polystyrene microparticles (Fig. 4a)

The following example shows the procedure for producing 5 micron polystyrene microparticles using the methodology described in this invention. The generation of the emulsion takes place using a capillary flow focussing device (D = 100 µm) with one single feeding end (D₀ = H = 150 µm). The device for capillary flow focussing is immersed in a stirred 1 % w/v PVA aqueous solution under stirring. Through the feeding tube we inject a 4 % w/v polystyrene dissolution (Aldrich, Mw =4.000-200.000) in ethyl acetate (Panreac Chemistry) with 1 mL/h flow rate. The chamber is pressurized by means of the introduction of water with a 3 mL/min continuous flow rate. The generated o/w emulsion is kept under stirring during 16 h at room temperature so the extraction/evaporation of the solvent takes place. Solid particles are centrifuged (Orto Alresa mod. Digicen 20, 4.000 rpm, 10 min), washed three times in water, lyophilized and stored at 4 °C.

The analysis of the microparticles was made using an optical microscope (Leica DM LS) and an image editor program (d_{average} 5,29 m, DS 0,509) and a scanning electron microscope (Philips XL30) (Fig.4a).

### EXAMPLE 2

### Preparation of 9 micron polystyrene microparticles (Fig. 4b)

The following example shows the procedure for producing 9 micron polystyrene microparticles using the methodology described in this invention. It uses the same device for capillary flow focussing as in example 1. The procedure for obtaining microparticles and composition of the fluids are the same as described in example 1, changing only the fluids injection conditions. The polymer dissolution is injected with 2 mL/h flow rate of and the water flow rate is 2 mL/min.

The analysis of the microparticles was made using an optical microscope (Leica DM LS) and an image editor program (d_{average} 9,28 m, DS 0,86) and a scanning electron microscope (Philips XL30) (Fig.4b).

### EXAMPLE 3

### Production of 13 micron polystyrene microparticles

The following example shows the procedure for producing 13 micron polystyrene microparticles using the methodology described in this invention. The generation of the emulsion takes place using a capillary flow focussing device (D = 200 µm) with one single feeding tube (D₀ = H = 150 µm). The device for capillary flow focussing is immersed in a 1 % w/v PVA aqueous dissolution under stirring. Through the feeding tube we inject a 4 % w/v polystyrene solution (Aldrich, Mw =4.000-200.000) in dichloromethane (Aldrich) with 3 mL/h flow rate. The chamber is pressurized by means of the introduction of water with a 4 mL/min continuous flow rate. The generated o/w emulsion is kept under stirring during 16 h at room temperature so the extraction/evaporation of the solvent takes place. Solid particles are centrifuged (Orto Alresa mod. Digicen 20, 4.000 rpm, 10 min), washed three times in water, dried by introducing it in boiling bath of water and stored at 4 °C.

The analysis of the microparticles was made using an optical microscope (Leica DM LS) and an image editor program (d_{average} 13,58 m, DS 1,65) and a scanning electron microscope (Philips XL30).

### EXAMPLE 4

### Production of polystyrene and rhodamine B fluorescent microparticles

The following example shows the procedure for producing 5 micron polystyrene microparticles using the methodology described in this invention. It uses the same device for capillary flow focussing and the same test conditions as in example 1. Here the fluids to be injected are homogeneous mixtures of a 4% w/v polystyrene dissolution (Aldrich, Mw = 4.000 - 200.000) in ethyl acetate (Panreac Chemistry) with diverse concentrations of rhodamine B.

The analysis of the microparticles was made using an optical microscope (Leica DM LS) and an image editor program (table I), and a fluorescency microscope (Leica DMR) (Fig. 5), a flow cytometer (FACScalibur, Becton Dickinson) and a scanning electron microscope (Philips XL30)

In table I we can see the reproducibility of the method.

**Table I. Size distribution of PS particles with rodhamine B**

| **[Rodhamine B] mM** | **D_{average} ± DS (µm)** | **CV (%)** |
|---|---|---|
| 0.006 | 5.29 ± 0.50 | 9.45 |
| 0.06 | 5.46 ± 0.61 | 11.23 |
| 0.6 | 5.29 ± 0.56 | 10.66 |

### EXAMPLE 5

### Production of polystyrene and fluorescein fluorescent microparticles

The following example shows the procedure for producing 5 micron fluorescent polystyrene microparticles using the methodology described in this invention. It uses the same device for capillary flow focussing and the same test conditions as in example 4 changing the fluorophore used. Here the fluids to be injected are homogeneous mixtures of a 4% w/v polystyrene dissolution (Aldrich, Mw = 4.000 - 200.000) in ethyl acetate (Panreac Chemistry) with diverse concentrations of fluorescein.

The analysis of the microparticles was made using an optical microscope (Leica DM LS) and an image editor program (table II), a fluorescency microscope (Leica DMR), a flow cytometer (FACScalibur, Becton Dickinson) and a scanning electron microscope (Philips XL30).

In Table II we can see the reproducibility of the method.

**Table II. Size distribution of PS particles with fluorescein**

| **[Fluorescein] mM** | **D_{average} ± DS(µm)** | **CV (%)** |
|---|---|---|
| 0.01 | 5.21 ± 0.55 | 9.99 |
| 0.1 | 5.32 ± 0.69 | 13.04 |
| 1 | 5.63 ± 0.64 | 11.38 |

### EXAMPLE 6

### Analysis and characterization of a mixture of different fluorescent particles obtained in the examples above

This example shows up that it is possible to distinguish different fluorescent microparticle populations, obtained by means of the capillary flow focussing process described in this invention, using habitual techniques of fluorescency analysis.

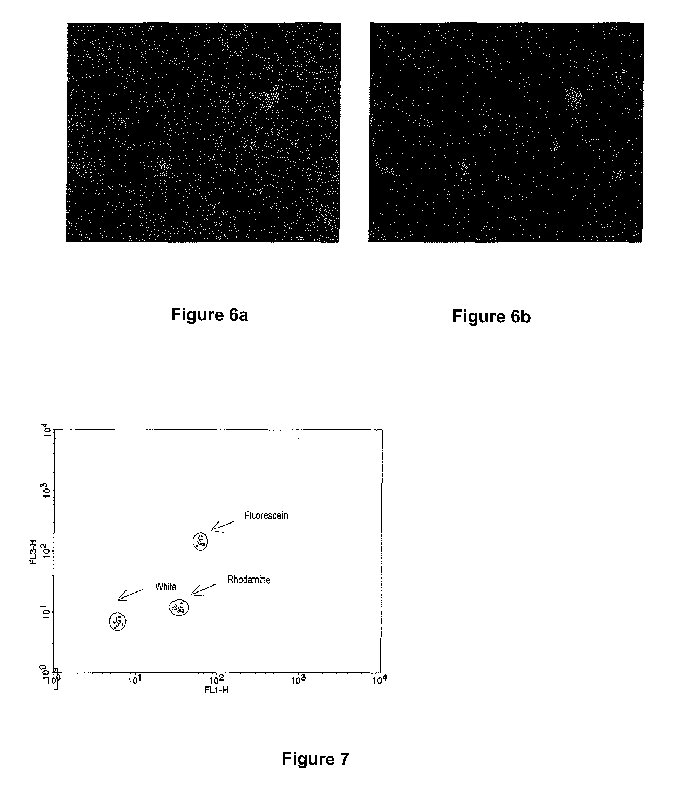

A mixture of 5 µm polystyrene microparticles, polystyrene with fluorescein 1 mM and polystyrene with rodhamine B 0,6 mM prepared according to the examples above, is suspended in water, they are sonicated during 5 minutes and analyzed in the fluorescency microscope (Leica DMR) using different filters (Fig. 6) and in a flow cytometer (FACScalibur, Becton Dickinson) (Fig. 7). Using both techniques we can distinguish clearly every population of particles in the mixture simultaneously.

## Claims

1. Procedure for the production of particles in the micro and nanometric range **characterized in that** the procedure includes the following steps:
• Introducing of two or more fluids in a capillary flow focussing device,
• Immersing of the capillary flow focussing device inside another fluid equally to or different to those introduced in the previous stage in the capillary flow focussing device,
• Generating of an emulsion where the continuous phase contains at least the fluid wherein the capillary flow focussing device was immersed, being the disperse phase constituted by the fluid or fluids focussed through the capillary flow focussing device, and
• Producing of the particles in the micro and nanometric range from the disperse phase generated in the previous stage, keeping those particles, with regard to the drops of the emulsion initially generated:
a) same morphology in the statistical size distribution, i.e. keeping the same momentums of higher order than the average normalized to the average, where this average can change due to the production process of the particle from the drop; and b) the same structure and morphology with regard to the relative inner structure of the different components.

2. Procedure for obtaining particles in the micro- and nanometric range, according to claim 1, **characterized in that** at least one of the focussed fluids in the capillary flow focussing device contains at least one solvent susceptible to be eliminated by means of extraction/evaporation to produce the particles of micro and nanometric size.

3. Procedure for obtaining particles in the micro- and nanometric range, according to claim 2, **characterized in that** the introduction of the fluids takes place by means of any method allowing the continuous supply of fluids without perturbations of the flow rate, in particular by means of compressors, pressurized chamber and volumetric pumps.

4. Procedure for obtaining particles in the micro- and nanometric range, according to claim 3, **characterized in that** the drops constituting the dispersed phase of the emulsion have a final diameter between 0,01 and 1000 µm, preferably between 0,01-200 µm and more preferably between 0,01-80 µm.

5. Procedure for obtaining particles in the micro- and nanometric range, according to claim 4, **characterized in that** the drops constituting the dispersed phase of the emulsion have a size distribution with a relative standard deviation of 10 to 30 %, preferably 3 to 10 % and more preferably less than 3%.

6. Procedure for obtaining particles in the micro- and nanometric range, according to claim 5, **characterized in that** during the introduction of the fluids in the capillary flow focussing device we apply to one or more fluids periodical and controlled external perturbations, in particular mechanical and acoustic perturbations, in order to enhance even more the production of particles with a more homogeneous size distribution.

7. Procedure for obtaining particles in the micro- and nanometric range, according to claim 6, **characterized in that** the applied fluids can be liquid or gases.

8. Procedure for obtaining particles in the micro- and nanometric range, according to claim 7, **characterized in that** all fluids are liquids.

9. Procedure for obtaining particles in the micro- and nanometric range, according to claim 8, **characterized in that** the fluids which are liquid can be simple liquids, mixtures, solutions, suspensions, emulsions, liquidized solids, etc. chosen in order to generate a stable microjet of the fluid or fluids focussed by the focussing fluid.

10. Procedure for obtaining particles in the micro- and nanometric range, according to claim 9, **characterized in that** the supplied fluid includes polymeric materials, silica, metals or ceramics, which constitute the matrix of the particles, and may additionally include other substances.

11. Procedure for obtaining particles in the micro- and nanometric range, according to claim 10, **characterized in that** those additional substances will result encapsulated in the obtained particles.

12. Procedure for obtaining particles in the micro- and nanometric range, according to claim 11, **characterized in that** the focussed fluid is preferably a dissolution, blending, suspension and/or an homogeneous emulsion of a polymeric material.

13. Procedure for obtaining particles in the micro- and nanometric range, according to claim 12, **characterized in that** the injected polymeric material can be synthetic or natural, soluble in water or in organic solvents.

14. Procedure for obtaining particles in the micro- and nanometric range, according to claim 13, **characterized in that** the polymeric material is selected preferably among the following: polyalcohols, polyacetals, polyethers, polyesters, [such as polylactic acid, polyglycolic acid, poly(caprolactone) and similar ones and their copolymers], polyorthoesters, polyanhydrides [such as polysebacic acid, polyfumaric acid, poly (carboxyphenoxy propane), poly(carboxyphenoxy hexane) and similar ones and their copolymers], polyaldehydes, polyketones, polycarbonates, poly(iminocarbonates), polyamides, polyimide, polyacrylates and their derivates and copolymers, poly(cyancrilates), polyurethanes, polystyrenes, polychlorides, polyfluorides, polyvinyl derivates, polyolefins, polyphosphates, poly(organic phosphacens), poly(anhydrides-co-imides), polysaccharides, and carbohydrates derivates, poly(aminoacid), polymers derived from macromolecules, and all derivates of the ones mentioned above and their copolymers.

15. Procedure for obtaining particles in the micro- and nanometric range, according to claim 14, **characterized in that** the polymeric material is preferably polystyrene and its derivates or copolymers.

16. Procedure for obtaining particles in the micro- and nanometric range, according to claim 12, **characterized in that** the applied polymeric materials have functional reactive groups that may react with any type of molecule containing the appropriate chemical functionality making possible the creation of one or more covalent bonds between particle and molecule.

17. Procedure for obtaining particles in the micro- and nanometric range, according to claim 16, **characterized in that** those reactive groups of the materials used are located in the surface of the drops exposed to the external environment.

18. Procedure for obtaining particles in the micro- and nanometric range, according to claim 11, **characterized in that** the encapsulated substances include preferably fluorescent material.

19. Procedure for obtaining particles in the micro- and nanometric range, according to claim 18, **characterized in that** as encapsulated fluorescent material can be included any type of material emitting a fluorescent signal, whether they are organic compounds, biomolecules, nanocrystals, nanoparticles, liquids, solids, etc. individually or as a mixture of several of those.

20. Procedure for obtaining particles in the micro- and nanometric range, according to claim 19, **characterized in that** the fluorescent material is encapsulated individually or in combination with others inside the same particle, so that it fulfils:
• have an excitement spectrum in the same range of wave lengths, and
• have an emission spectrum that enables to distinguish them when used simultaneously.

21. Procedure for obtaining particles in the micro- and nanometric range, according to claim 20, **characterized in that** the injected fluid containing the fluorescent material constitutes a homogeneous mixture (solution, suspension, emulsion, etc.) with a known composition during the complete generation process of the emulsion.

22. Procedure for obtaining particles in the micro- and nanometric range, according to claim 10, **characterized in that** the additional substances present in the fluids include molecules of biological interest, preferably peptides, oligonucleotides, nucleic acids, PNAs, LNAs, proteins, glycoproteins, lipids, phospholipids, carbohydrates, oligosaccharides and mixtures of those.

23. Procedure for obtaining particles in the micro- and nanometric range, as in any of the preceding claims, **characterized in that** the fluid wherein the capillary Flow Focussing device is immersed is a liquid, of aqueous or organic nature, which allows the generation of the emulsion in which the fluid or fluids constituting the dispersed phase keep an emulsion drop size equally to the one produced by the dissociation of the capillary jet.

24. Procedure for obtaining particles in the micro- and nanometric range, according to claim 23, **characterized in that** the fluid wherein the capillary Flow Focussing device is immersed is a liquid that may, as an option, contain substances in dissolution which enhance the maintenance of the uniformity and homogeneity of the emulsion during the complete generation process and extraction/evaporation of the solvent, in particular emulsifiers or tensioactives.

25. Procedure for obtaining particles in the micro- and nanometric range, according to claim 24, **characterized in that** the particles obtained can be solid, hollow or porous.

26. Procedure for obtaining particles in the micro- and nanometric range, according to claim 25, **characterized in that** the particles have a homogeneous matrix.

27. Procedure for obtaining particles in the micro- and nanometric range, according to claim 25, **characterized in that** the particles have several/different layers.

28. Procedure for obtaining particles in the micro- and nanometric range, according to claims 26 or 27, **characterized in that** the particles have in their surface functional reactive groups that can create covalent links with molecules of biological interest, preferably peptides, oligonucleotides, nucleic acids, PNAs, LNAs, proteins, glycoproteins, lipids, phospholipids, carbohydrates, oligosaccharides and mixtures of those.

29. Device for obtaining particles in the micro- and nanometric range by means of a procedure, according to claim 28, **characterized in that** it is a capillary Flow Focussing device comprising:
• a chamber pressurized by means of the continuous supply of a fluid which has one exit orifice on its wall, and
• a feeding source of fluids located inside that chamber.

30. Device for obtaining particles in the micro- and nanometric range, according to claim 29, **characterized in that** the feeding source located inside the chamber is constituted by a single capillary tube placed in front of the hole existent **in that** chamber wall.

31. Device for obtaining particles in the micro- and nanometric range by means of a procedure, according to claim 28, **characterized in that** it is a capillary Flow Focussing device comprising:
• a chamber pressurized by means of the continuous supply of a fluid which has multiple exit orifices to the outside on its wall, and
• one fluids feeding source of the fluids constituted by multiple feeding tubes located inside the pressurized chamber, each of them constituted by a single capillary tube placed in front of one of the multiple exit holes located in the wall of that chamber.

32. Device for obtaining particles in the micro- and nanometric range, according to claim 29, **characterized in that** the fluids feeding source located inside the chamber is constituted by a bundle of capillary tubes located concentrically to each other and place in front of the exit orifice of that chamber.

33. Device for obtaining particles in the micro- and nanometric range, according to claim 31, **characterised in that** each of the multiple feeding ends located inside the pressurized chamber is constituted by a bundle of concentric capillaries placed in front of one of the multiple holes located in the wall of that chamber.

34. Device for obtaining particles in the micro- and nanometric range, according to claim 33, **characterised in that** each feeding end is placed in front of a single exit hole of those located on the chamber wall.

35. Device for obtaining particles in the micro- and nanometric range, according to claims 32 or 33, **characterized in that** the exit end of the internal capillary, part of the feeding source located inside the chamber, and the exit hole of that chamber, are placed preferably at a distance between zero and three times the value of the inner diameter of the external capillary, part of the feeding source.

36. Device for obtaining particles in the micro- and nanometric range, according to claim 35, **characterized in that** the relative distance between the exit ends of the concentric tubes, part of the feeding source, can change, and preferably the exit end of the internal capillary tube does not enter the adjacent external capillary tube a distance higher than the value of the external tube inner diameter.

37. Device for obtaining particles in the micro- and nanometric range, according to claim 36, **characterized in that** preferably the exit end of the internal capillary tube does not stand out from the adjacent external capillary tube a distance higher than twice the value of its inner diameter.

38. Device for obtaining particles in the micro- and nanometric range, as in one of claims 29-37, **characterized in that** the capillaries that constitute the end of the feeding source which are inside the pressurized chamber have preferably 0,002 to 2 mm inner diameter, and more preferably between 0,01 and 0,30 mm.

39. Device for obtaining particles in the micro- and nanometric range, according to claim 38, **characterized in that** the exit holes of the chamber have preferably an inner diameter between 0,002 and 2 mm, and more preferably between 0,01 and 0,25 mm.

40. Device for obtaining particles in the micro- and nanometric range, according to claim 39, **characterized in that** each exit hole of the chamber and its corresponding exit end of the fluids feeding source located inside the chamber are separated a distance between 0,01 and 2 mm, preferably between 0,2 and 0,6 mm.

41. Device for obtaining particles in the micro- and nanometric range, according to claim 40, **characterized in that** the feeding sources are preferably capillary tubes, porous media or any other medium able to distribute an homogeneous flow rate among different feeding ends.

42. Device for obtaining particles in the micro- and nanometric range, according to claim 41, **characterized in that** the inner diameters of the capillary tubes constituting the feeding source, the diameter of the exit hole of the chamber and the distance between them can be changed and adjusted in order to obtain interaction conditions between the fluids leading to a stable capillary microjet inside the laminar flow.

43. Device for obtaining particles in the micro- and nanometric range, according to claims 42, **characterized in that** it can be manufactured in multiple materials, preferably metal, plastic, ceramics, glass.

44. Use of the particles produced by means of a procedure as in one of claims 1-28, as calibration standards.

45. Use of the particles produced by means of a procedure as in one of claims 1-28, in pharmaceutical and biomedical applications, preferably encapsulation and delivery of drugs, cells and microorganisms encapsulation, and diagnosis and clinical analysis.

46. Use of the particles produced by means of a procedure as in one of claims 1-28, to form arrays of codified particles with fluorescent material and modified in their surface with compounds of biological interest.
